Europäisches Patentamt

European Patent Office

Office européen des brevets

(11) Publication number: 0 402 078
A2

# EUROPEAN PATENT APPLICATION

(21) Application number: 90306054.9

(22) Date of filing: 04.06.90

(51) Int. Cl.⁵: A61K 31/315, A61K 33/30,
A61K 9/02

(30) Priority: 06.06.89 US 362058

(43) Date of publication of application:
12.12.90 Bulletin 90/50

(84) Designated Contracting States:
AT BE CH DE DK FR GB IT LI LU NL SE

(71) Applicant: Kelly, Patrick Daniel
33 Berry Oaks
St. Louis, Missouri 63122(US)

(72) Inventor: Kelly, Patrick Daniel
33 Berry Oaks
St. Louis, Missouri 63122(US)

(74) Representative: Woods, Geoffrey Corlett et al
J.A. KEMP & CO. 14 South Square Gray's Inn
London WC1R 5EU(GB)

(54) Sexual lubricants containing zinc as an anti-viral agent.

(57) This invention relates to the use of zinc as an anti-viral agent in scxual lubricants. A selected zinc compound which has antiviral properties, and which does not irritate the skin or mucous membranes, is provided in a sexual lubricant such as a water-soluble gel. The lubricant is spread on the surface of the penis, or inside the vagina, before sexual intercourse. The zinc can serve as a preventive agent to inhibit the transmission of genital herpes through sexual contact, and may also help to reduce the spread of other sexually transmitted viral diseases such as AIDS. Such lubricants can be used in addition to a condom for maximal protection, or without a condom for a lesser yet still significant level of protection compared to unprotected intercourse. A preferred zinc salt which does not cause irritation during or after sexual intercourse comprises zinc gluconate, the salt of a carboxylic acid derivative of glucose. Other suitable zinc salts can be prepared from carboxylic acid derivatives or other oxidation products of other sugars.

## SEXUAL LUBRICANTS CONTAINING ZINC AS AN ANTI-VIRAL AGENT

This invention is in the fields of biochemistry, pharmacology, and anti-viral agents.

There is a major need for methods to prevent the spread of viral diseases which are transmitted through sexual contact. The most widespread sexually transmitted viral disease is genital herpes, an incurable disease usually caused by a virus referred to as herpes simplex virus type 2 (HSV-2). Herpes simplex virus type 1 (HSV-1) is usually associated with cold sores on the mouth and gums; however, HSV-1 has also been isolated from genital lesions while HSV-2 has been isolated from cold sores.

Genital herpes, in addition to causing painful lesions, poses a serious health threat. It can cause malignant transformation in animal and human cells, and has been linked to an increased risk of cervical and vulvar cancer in women. The virus can also infect babies during birth, causing neonatal herpes, which is 80% fatal and can cause severe health problems or deformation if the baby survives.

Herpes lesions on the genitals are also believed to provide viral entry and exit sites which increase the risk of becoming infected with other viral diseases such as AIDS during intercourse. If someone infected with herpes has intercourse with someone who has the human immunodeficiency virus (HIV), the person with herpes is more likely than a non-herpetic would be to contract AIDS as a result. In addition, for people infected with both herpes and HIV (as is the case with many prostitutes), herpes lesions can provide exit points for the HIV virus to reach sexual partners in higher concentrations during intercourse. Due to both factors, any method for preventing the spread of genital herpes is also likely to help reduce the spread of AIDS.

The need for methods for reducing the spread of herpes and AIDS are especially acute in view of the difficulties that have been encountered in trying to develop effective vaccines against either virus. Despite intensive effort, there has been very little progress toward developing successful vaccines against either virus, for several reasons. The capsid particles for both viruses are glycosylated; their surfaces are coated by sugar molecules that can prevent a host's immune cells from contacting the viral proteins, recognizing them as foreign, and generating antibodies which can bind to them. In addition, both viruses can dwell for long periods in nerve cells that are inaccessible to antibodies. HIV also mutates very rapidly; the amount of variation in a single patient is likely to be enough to render antibodies ineffective against the virus (see Marx 1988; a list of references with complete citations is contained near the end of this application, before the claims). For additional general information on herpes simplex viruses, see, e.g., Mindel 1989. For more information on human immunodeficiency virus and AIDS, see, e.g., Wormser et al 1987.

There are several other sexually transmitted viruses that are also medically and epidemiologically important, including hepatitis, papilloma, and Epstein-Barr viruses. In addition to causing their own adverse symptoms, which can be severe, all three types of virus have been linked to cancer.

The most widely recommended protective measures to prevent the spread of herpes, AIDS, or other sexually transmitted viruses involve impermeable barriers to prevent any contact of body fluids with the skin, or with mucous membranes exposed in the mouth, vagina, urethra, etc. "Barrier protection" in the context of sexual intercourse involves the use of condoms; other types of barrier devices, including diaphragms and cervical caps, may protect the uterus, thereby possibly reducing the risk of infection, but they do not protect the rest of the vagina, or the penis.

Even though many health care professionals and other people and the scientific, medical, and mass media have repeatedly urged all sexually active, non-monogamous people to use condoms, many people at risk either fail or deliberately refuse to follow that advice. In addition, condoms do not offer complete assurance of safety, since they can break. Condoms can also tear out pubic hairs, thereby creating tiny injuries to the epidermis which may serve as entry routes for viruses.

There is, therefore, an urgent need for additional methods and agents which can help prevent the spread of sexually transmitted viruses. As used herein, "anti-viral" refers to any agent or method that can inhibit the replication or transmission of a virus in an exposed or infected person or animal, regardless of which stage or step of the viral cycle is inhibited (replication within a cell, release from an infected cell, transmission across a membrane or within a body, etc.).

Before the AIDS epidemic began, several studies indicated that zinc ions can inhibit the replication of various different types of virus. Those studies include Gordon et al 1975 (herpes virus), Bracha et al 1976 (sindbis virus), Firpo et al 1979 (foot and mouth disease virus), Zaslavsky et al 1979 (vaccinia viruses), and Sharma et al 1985 (aphthovirus).

The effects of zinc on rhinoviruses (a class of viruses which cause common colds) have also been studied, but the results have been inconsistent. Korant et al 1976A and 1976B reported that zinc ions can inhibit the replication of some but not all rhinoviruses. Eby et al 1984 reported that in a double-blind test, zinc gluconate tablets, when chewed up and allowed to soak in the mouth, reduced cold symptoms. Eby also obtained US patent 4,503,070 (1985) on the use of zinc gluconate lozenges to treat cold symptoms. That patent was followed by US patent 4,684,528 (Godfrey 1987) on flavor-masked zinc lozenges.

Those positive reports provoked a series of rhinovirus studies which led to negative results, including Douglas et al 1987, Farr et al 1987, and Geist et al 1987. Some of the factors which contributed to the confusing and inconsistent results include concentration and chelation, which are discussed with a rather disappointing lack of clarity in Godfrey et al 1988. That exchange of letters makes it clear that the people studying the effects of zinc ions on rhinoviruses are divided on the question of whether it works, and those who claim that it works cannot explain why. The most extensive study done to date, Al-Nakib et al 1987, stated, "We conclude that zinc gluconate lozenges...have a significant effect on the signs and symptoms of colds caused by rhinoviruses, although the mechanism of action remains obscure."

One of the factors that may be contributing to the confusion and disagreements over rhinoviruses, but which did not receive adequate attention in the exchange of letters in Godfrey et al 1988, is that some strains of virus may be resistant to inhibition by zinc, in a manner analogous to the way some bacteria are resistant to antibiotics. For example, the inventor has used zinc lozenges roughly twenty times to fight colds, since learning about Eby's patent 4,503,070. In most cases, he found that zinc was highly effective and either prevented the cold entirely, or greatly reduced its severity. However, in one or two specific instances, it seemed to have little or no effect.

Another factor that may have contributed to the confusion over zinc and rhinoviruses involves the apparent need for direct contact between the zinc ions and the virus. On a couple of occasions when the inventor chewed zinc gluconate tablets to fight colds, he ended up with a severe runny nose and soreness in the lower throat, but with no adverse symptoms inside the mouth. Those results suggest that the anti-viral action of zinc depends upon direct contact of the zinc ions with the viral particles. However, several of the rhinovirus studies tried to evaluate zinc by applying it in the mouth and then determining whether it could prevent a cold that was induced by injecting viral particles into the nose via nasal sprays. In addition, some of the assays for effectiveness involved nasal washings, to determine how many viable virus particles remained in the nasal sinuses after zinc was applied to the mouth. If indeed zinc requires direct contact with viruses to be effective, then both approaches were apparently too indirect; they are analogous to testing an antibiotic ointment against a bacterial infection by applying the ointment several inches away from a skin cut.

At least five different research teams' have reported that certain salts of zinc, used by some researchers in combination with other active agents such as tannic acid or heparin, is effective as a topical agent in treating genital herpes lesions. A team in Missouri led by Dr. Mostafa Fahim reported that guinea pigs and humans (both male and female) suffering from genital herpes improved substantially when treated by a combination of ultrasound and an ointment (which they gave the tradename "Herpigon") containing 30% urea, 3% zinc sulfate, 2% tannic acid, and 65% HEB cream; see Fahim et al 1978, Brawner et al 1979, Fahim et al 1980a, and Fahim et al 1980b.

In Arizona, a team led by Dr. Patrick Tennican reported that female mice which had been previously inoculated with herpes virus, and female humans seeking treatment for genital herpes, improved when a collagen sponge containing zinc sulfate was placed inside the vagina; see Tennican et al 1979 and Tennican et al 1980.

In Israel, Dr. Asher Wabha reported that 18 patients, four of whom had genital herpes, showed improvement after being treated with zinc sulfate in water; see Wahba et al 1980.

In Texas, George Eby and Dr. William Halcomb reported that aqueous solutions of zinc gluconate accelerated recovery in four human females suffering from herpes; see Eby et al 1985.

And in Switzerland, Bohumir Lukas et al disclosed that a mixture of zinc sulfate and heparin reduced herpes infections in tissue culture tests and in guinea pigs; see US patent 4,465,666, assigned to Ciba-Geigy.

There have also been reports that zinc solutions help people suffering from non-genital herpes, including cold sore lesions in the mouth (see Wahba et al 1980 and Brody et al 1981) and keratitis lesions in the eyes (see DeRoeth et al 1963).

The work by Lukas et al is a good example of the apparent attitude of most medical researchers toward the use of zinc to combat herpes infections. In column 3 of patent 4,465,666, Lukas et al reported that zinc sulfate, when mixed with a gel carrier and applied to guinea pigs three days after the guinea pigs were infected with herpes viruses, reduced the number of guinea pigs suffering from observable symptoms. As described by Lukas, about 50% of the animals treated with the gel alone were free of herpes symptoms, but if zinc was added to the gel, then about 70% of the animals were free of symptoms. However, Lukas et al criticized that result and pointed out the additional improvement that could be obtained by adding both zinc and heparin to the gel. When both compounds were used, more than 90% of the animals were free of symptoms.

That result indicates that heparin, when used jointly with zinc, provides a more effective treatment than zinc alone for animals that were previously infected with herpes. However, heparin is a powerful anti-coagulant; it prevents the blood from clotting normally. Therefore, even though it might be used safely under the supervision of a physician for several days (the typical duration of a herpes flare-up that is properly treated), it would be highly inadvisable to administer heparin to anyone on a frequent basis (such as every night for weeks or months), or to sell it for use as an over-the-counter non-prescription drug. It is clear, therefore, that the inventors of patent 4,465,666 did not anticipate the potential of zinc, without heparin, in a form that can be used frequently to provide an anti-viral protective agent for people who are not yet infected by herpes.

All of the reports cited above which involve herpes involve the use of zinc compounds to treat herpes lesions, or to prevent the occurrence of active outbreaks, in animals or people who are already infected with herpes virus. To the best of the Applicant's knowledge, no one has ever publicly suggested the use of zinc as a preventive agent to inhibit the transmission of herpes to someone who is not already infected.

In a patent application filed under the Patent Cooperation Treaty, number WO 8702246, William Sergio of Florida suggested that the risk of infection with AIDS might be reduced by topical administration of compounds which generate anions having charges greater than one. Sergio's reference to anions appears to be mistaken, since an anion is a negatively charged ion, while zinc is released from a zinc salt as a cation (positively charged). Sergio states that his preferred salts are zinc phosphonoformate and/or zinc tungstate. However, it is likely that the use of either of those compounds as a sexual lubricant would cause severe irritation to the mucous membranes and perhaps to the epidermis as well, and might also cause various other adverse effects. His suggestion concerning zinc tungstate appears to be based on the tungsten-containing compound HPA-23, which, as Sergio concedes elsewhere, has "serious side effects" (Sergio 1988).

There remains, therefore, a need for a sexual lubricant which is non-toxic and non-irritating to highly sensitive mucous membranes even when vigorously rubbed in for prolonged periods of time, yet which is also effective as an anti-viral agent.

This invention relates to the use of zinc as an anti-viral agent in sexual lubricants. A selected zinc compound which has anti-viral properties, but which does not irritate the skin or mucous membranes, is provided in a sexual lubricant such as a water-soluble gel. The lubricant is spread on the surface of the penis, or inside the vagina, before intercourse. The zinc can serve as a preventive agent to inhibit the transmission of genital herpes through sexual contact, and may also help to reduce the spread of other sexually transmitted viral diseases such as AIDS, hepatitis, and papilloma viruses. Such lubricants can be used in addition to a condom for maximal protection, or without a condom for a lesser yet still significant level of protection compared to unprotected intercourse. A preferred zinc salt which does not cause irritation during or after sexual intercourse comprises zinc gluconate, the salt of a carboxylic acid derivative of glucose. Other suitable zinc salts can be prepared from carboxylic acid derivatives or other oxidation products of other sugars.

In one preferred embodiment of this invention, a selected zinc compound which has anti-viral properties is added to a sexual lubricant. The lubricant is then spread on the surface of the penis, or inside and around the vagina, before sexual intercourse. It can be used in addition to a condom or other barrier device for maximal protection, or without a condom for a lesser yet still significant level of increased protection compared to unprotected intercourse.

As used herein, the term "sexual lubricant" refers to any gel, emulsion, lotion, ointment, or other fluidized substance which is spread on the sexual organs of the male or female before or during intercourse.

One water-soluble gel which is widely used as a sexual lubricant is sold over-the-counter by Johnson and Johnson (New Brunswick, NJ) under the tradename "K-Y Lubricating Jelly." It contains purified water, glycerine as a lubricant, hydroxyethylcellulose as the thickening agent, glucono-delta-lactate to prevent crystallization, chlorhexidine gluconate as a preservative, and sodium hydroxide to reduce the acidity. A generic version which appears to have the same ingredients is sold by Walgreens. A modified form of KY Lubricating Jelly sold by Johnson and Johnson contains propylene glycol residuals, which are a by-product of propylene oxide, a sterilizing agent.

Various other water-soluble gels can also be used. For example, several other derivatives of cellulose, such as methylcellulose or ethylcellulose, can be added to water to form a gel which is viscous (slow-flowing) and hydrophilic (water-soluble). Other derivatives of cellulose, such as carboxymethyl-cellulose or microcrystalline cellulose, may also be suitable, as can be determined through routine experimentation as described below.

For most people, hydrophobic substances (i.e., substances which are not soluble in water) are not preferred as sexual lubricants, since they cannot be washed off easily, and because some of them (such as petrolatum) can damage some types of condoms. However, it was stated in Shilts 1987 that a certain vegetable shortening (sold under the trademark Crisco, by Proctor and Gamble, Cincinnati, Ohio) is used as a sexual lubricant by some male homosexuals who engage in frequent anal intercourse, presumably because it does not rub off easily and remains in place longer, offering prolonged lubrication. Accordingly, it may be possible to use hydrophobic substances for the purpose of this invention, as a carrier for zinc ions. However, any such hydrophobic substance should be tested *in vivo* to ensure that it does not sequester the zinc ions in microdroplets or otherwise render it ineffective as an anti-viral agent.

Various ointment and lotion formulations are known to those skilled in the art; several are described in detail in the *U.S. Pharmacopeia* and in various texts on cosmetics. In addition, various emulsions (viscous mixtures of hydrophobic and hydrophilic substances, usually in the form of microscopic droplets of one liquid suspended in the other liquid) are also known to those skilled in the art; see, e.g., Lieberman et al 1988, Becher et al 1965, and Robinson et al 1987. Such ointments, lotions, and emulsions can be used to carry zinc for the purpose of this invention, if no significant quantities of compounds which chelate the zinc are present. However, ointments and lotions which contain alcohols or other substances which irritate the genitals, and emulsions contain emulsifying agents which irritate the genitals, should be avoided.

If desired, compounds such as preservatives, coloring agents, and fragrances can be added to the sexual lubricants described herein, provided that (1) they do not chelate the zinc ions or otherwise interfere with the anti-viral activity of the selected zinc compound, and (2) they do not irritate or have other adverse effects on the genitals.

Zinc salts which are suitable for use as described herein must have two characteristics. First, they must have a substantial degree of anti-viral activity. Such activity can be initially tested using *in vitro* assays, as described below, and can be confirmed with respect to specific formulations (such as a specific zinc salt in a hydrophilic gel, hydrophobic cream, emulsion, etc.) using animal tests involving mice or guinea pigs, which are highly susceptible to herpes.

Second, any zinc salt used as described herein must be non-irritating to the genital skin and to the mucous membranes inside the vagina. This can be determined using a sequence of tests involving volunteers, who preferably should have some understanding of the process, such as medical students, hospital interns, nurses, graduate students in biology or chemistry, and their spouses. A preferred sequence of tests is as follows.

In the first test, a selected zinc salt is first tested on the relatively hairless portion of the forearm, using a mortar and pestle to grind the zinc salt into a fine powder, then using distilled and deionized water to dissolve or suspend the salt in solution and spread it evenly across a patch of skin. If the zinc salt does not cause irritation after being left in place for an hour or more, the second test involves spreading it upon the male genitals. The first test on the male genitals should involve a passive test; the zinc compound is applied gently and allowed to remain, without rubbing it in, for a period such as an hour. If it causes no irritation in that test, the third test involves mixing the zinc salt with a lubricant such as a gel, applying it to the male genitals, and rubbing it in for several minutes in a manner that simulates the rubbing motion of intercourse. If it causes no irritation in that test, it can be applied gently (dissolved in either water or gel) to a female volunteer, to the mucous membranes that are near the opening of the vaginal canal which can be reached without deep penetration. The woman should have a douche readily available before the test begins, to rinse out the substance if it begins to cause irritation. If the zinc compound causes no irritation in that type of test, it can be applied to the deeper areas in the vagina, preferably using a finger during the first such test to apply it and to rub it in gently. If no irritation results, it can be tested during actual intercourse. During the first test involving intercourse, the zinc compound preferably should be applied and tested only after a first act of coitus has been completed, so that if irritation does result, the rest of the evening won't be ruined and the partners won't be left in a state of sexual frustration, which exaggerates any feelings of sexual irritation.

People vary in their skin sensitivity; for example, some people are easily sunburned while others are highly tolerant of direct sunlight. Therefore, as used herein, references to "non-irritating" zinc salts or formulations refer to salts or formulations that do not cause irritation in at least some people. Such formulations can be used by such people regardless of whether they may cause irritation in other people with more sensitive skin or membranes.

A preferred zinc compound for use as described herein comprises zinc gluconate, which was purchased from a commercial supplier and tested using the series of tests described above. It did not cause any significant irritation during intercourse using K-Y Lubricating Jelly as the carrier substance. Zinc gluconate is a physiologically acceptable salt of zinc, which is in turn an essential mineral. Zinc gluconate tablets (23 mg) are sold over the counter as mineral supplements at Walgreens and other drug stores. It should also be noted that zinc is an essential mineral and is required for proper functioning of the immune system (see, e.g., Bach 1981). Therefore, if zinc as used herein permeates through the skin or vaginal membranes into the bloodstream, it may have an additional beneficial effect of helping the immune system function properly, especially in people who suffer from low levels of zinc (see Weiner 1984).

By contrast, zinc chloride caused a high level of irritation in the forearm test, and was not tested further. Zinc sulfate did not cause any irritation in the forearm or male genital tests, but it caused substantial irritation when applied to the shallow areas of a vagina and was not tested during intercourse. All three salts were tested at relatively high concentrations, to provide a cautious appraisal; if zinc gluconate caused no irritation at a relatively high concentration, then it should not cause irritation at lower concentrations.

Zinc oxide was also tested and confirmed to be non-irritating in the vagina during intercourse. Zinc oxide is the main active ingredient in Desitin ointment, which is spread on babies bottoms and genitals to cure diaper rash, so it was expected to be non-irritating. However, unlike zinc gluconate and zinc sulfate, the Applicant is not aware of any published reports indicating that zinc oxide has anti-viral properties. Its anti-viral activity can be determined through routine experimentation as described below, and if zinc oxide is shown to have anti-viral properties, it will be suitable for use according to this invention.

Various other salts of zinc, particularly organic salts formed by combining zinc with various organic molecules having carboxylic acid groups, are preferred candidates for evaluation to determine whether they are both non-irritating and anti-virally effective. Zinc gluconate is the salt that results from combining one molecule of zinc (which is divalent) with two molecules of gluconic acid ($C_5H_6(OH)_6COOH$), which is an oxidation product of glucose, a sugar molecule with six carbon atoms. Zinc salts formed by combining zinc with carboxylic acid derivatives or other oxidation products of various other carbohydrates (such as other sugar molecules with more than about four or five carbon atoms) are therefore preferred candidates for testing to evaluate their suitability for use as described herein. Any such molecule which has a higher dissociation constant at physiological pH than zinc gluconate, and which is tested and shown to be non-irritating during intercourse, is especially preferred, since it will release more zinc ions than zinc gluconate.

Zinc compounds which display low but significant and adverse levels of irritation when mixed with an aqueous gel can be tested to determine whether the irritation can be lowered to acceptable levels, using two different method. The first method involves lowering the concentration of the zinc compound in the aqueous solution. The second method involves the use of other carrier substances such as ointments and emulsions, many of which are specifically formulated to mask the irritating effects of certain active ingredients in various formulations. For example, many cosmetic formulations, shampoos, and other topical mixtures contain various types of alcohol or detergent which would severely irritate the skin if applied in concentrated form, but which are quite acceptable if present in low quantities in creams or ointments that contain other ingredients designed to soothe the skin.

Any zinc salt or other zinc compound can be tested for anti-viral activity using *in vitro* tests. *In vitro* tests involve cell cultures rather than intact animals; see, e.g., Gordon et al 1975. If an *in vitro* assay involves anchorage-dependent cells (such as fibroblasts or other skin cells), the typical steps involve (1) growing a layer of cells on a solid or semi-solid nutrient such as agar, to form a "lawn" of cells across the surface of the culture plate; (2) spreading a liquid solution containing viral particles (such as herpes simplex type I or II) across the layer of cells; (3) incubating the cells and viruses for an appropriate period of time, and (4) determining how many clusters of cells were killed by the viruses, as evidenced by rounded zones ("plaques") that have been cleared of viable cells. The result of such a test is usually expressed in terms of "plaque-forming" viruses per volume of solution. If a certain zinc compound reduces the number of plaques (i.e., if it allows more cells to survive), then that compound has *in vitro* anti-viral activity.

*In vitro* assays involving cells that are not anchorage-dependent (such as white blood cells) are done in an analogous manner, using liquid nutrients and viral "titers" (i.e., solutions with varying concentrations of viruses). Instead of measuring immobile plaques, the viable cells that remain in the solution after the viral challenge can be measured. Most titer assays, rather than counting the number of viable cells that remain in a test tube at the end of the challenge, are designed to determine the concentration of viruses which effectively wipe out a cell culture. If a specific zinc compound allows a cell culture to withstand a concentration of viruses higher than the cells can withstand without the zinc compound, then the compound has *in vitro* anti-viral activity.

Some viruses infect only certain types of cells; in general, herpes viruses infect anchorage-dependent cells such as skin cells and nerve cells, while the AIDS virus infects only certain types of blood cells. Therefore, a suitable combination of cells and viruses must be used in any specific test.

For the purposes of this invention, a zinc compound is regarded as having *in vitro* anti-viral activity if it significantly inhibits one or more types of sexually transmitted virus. It is not necessary for a zinc compound to inhibit all types of sexually transmitted viruses in order to be useful as described herein to help reduce the spread of that type of virus. Similarly, a sexual lubricant containing zinc is regarded as having anti-viral activity if it inhibits the replication, recurrence, or transmission of one or more types of sexually transmitted virus in *in vivo* tests. Even if an anti-viral sexual lubricant directly inhibits only one type of herpes virus, it is still useful in two different respects. First, it can directly inhibit the spread of that particular virus. And second, it can indirectly help reduce the spread of AIDS, by reducing, in a large population of people at risk, the number of people who suffer from herpes lesions. Such lesions serve as entry or exit ports for the AIDS virus during intercourse and therefore increase the spread of AIDS among people exposed to the AIDS virus.

Any zinc compound that has been shown to be anti-virally effective in *in vitro* assays, and which causes low levels of skin and mucous membrane irritation in tests as described above, is a good candidate for screening using *in vivo* tests on lab animals after it is mixed with a sexual lubricant as described herein. Alternately, several zinc compounds (including zinc gluconate) have been shown to provide substantial relief to people suffering from herpes infections. Those compounds have been shown to have anti-viral activity when mixed with aqueous solutions, and they can be used as described herein in aqueous lubricants such as gels. However, before being mixed with any hydrophobic carrier or emulsion, they should be tested for anti-viral activity in that particular carrier substance, using lab animals.

Animal tests involving infection through the genitals (to simulate sexual transmission) are normally done by inserting cell-free viral solutions, or infected cells containing viruses, into the vaginas of female animals or into the urethral mucosa of male animals. Tests involving herpes virus are commonly done on mice and guinea pigs; see, e.g., Tennican et al 1980. Tests involving the AIDS virus (HIV) are presently done mostly on monkeys and apes (see, e.g., Miller et al 1989), since most other animals are not susceptible to HIV infection. However, genetically engineered animals such as mice have been developed which carry human-derived T-cells, to enable researchers to perform tests involving the HIV virus on those animals. The tests described herein can be performed on any animal species or strain which is susceptible to a specific type of sexually transmitted virus being tested.

Alternately, it is possible to determine anti-viral effects in tests involving humans who are already infected by a certain type of sexually transmitted virus. Such tests would involve contacting the genitals with zinc-containing lubricant for a desired period of time, such as ten or twenty minutes, preferably while massaging the lubricant into the genitalia, to simulate the motions of intercourse. Subsequently, genital tissue or sexual fluids are sampled to determine whether the number of infective viral particles is reduced compared to untreated control subjects. Alternately, in the case of herpes, the treatment can be performed on people suffering from active outbreaks of lesions, preferably during the "rising red" phase of the outbreak. The subjects are monitored after the treatment to determine whether the lesions caused less pain, disappeared more rapidly, and recurred less frequently compared to untreated control groups. If a specific formulation containing a zinc compound and a sexual lubricant is shown to be effective in reducing the severity, duration, or recurrence of herpes outbreaks, then that formulation has *in vivo* anti-viral activity. Even though it will not offer completely reliable protection against becoming infected, it can reduce the number of people who contract sexually transmitted viral diseases after sexual intercourse with infected people.

Anyone buying such a lubricant should be clearly warned that the lubricant does not offer completely effective and reliable protection against infection. Nevertheless, in the absence of effective vaccines or cures for herpes or AIDS, most rational people who are sexually active and non-monogamous would prefer to take the precaution of using a lubricant shown to have anti-viral activity.

In an alternate preferred embodiment of this invention, zinc can be included in a sexual lubricant which also contains one or more other active agents. For example, US patent 4,507,281 (invented by Asculai and Rapp, assigned to Exovir, Inc. of Great Neck, New York) described a composition for treating herpes infections, containing interferon and "an anti-viral surface active agent" such as nonoxynol. As further described in Asculai et al 1978 and in US patents 4,020,183 and 4,139,630 (both by Asculai et al), non-ionic surface active agents with ether or amide linkages between the hydrophilic and hydrophobic portions of the molecule rapidly reduce the infectivity of herpes simplex viruses. Nonoxynol is sold without a prescription, for use during intercourse as a spermicidal contraceptive; it causes low levels of genital irritation in most people. It can be used in combination with zinc ions as described herein, to provide a doubly effective anti-viral sexual lubricant.

Similarly, an ointment sold in the United States under the generic name "Triple Antibiotic" contains polymyxin, bacitracin, and neomycin. Other ointments, which often require a prescription for purchase, contain beta-lactam antibiotics such as penicillin or ampicillin. Sexual lubricants which contain an anti-viral zinc compound and one or more such bacteriocidal compounds can inhibit the spread of viral diseases as well as non-viral diseases such as gonorrhea and syphilis.

Those skilled in the art will recognize, or may ascertain using routine experimentation, various equivalents to the specific embodiments of the invention described herein. Such equivalents are within the scope of the invention, which is limited solely by the claims below.

**EXAMPLES**

EXAMPLE 1: ZINC GLUCONATE

Zinc gluconate was purchased from Ruger Chemical Company (Irvington, New Jersey). It came in the form of a white powder interspersed with small hard granules. To remove the abrasive granules, it was ground into a fine powder using a mortar and pestle for several minutes when dry and again after the gel was added. Approximately 0.5 gram of zinc gluconate was used with about 15 milliliters of K-Y LUbricating Jelly. Although the mixture displayed a very slight roughness when rubbed hard between the forefinger and thumb, no abrasion was noticeable by either partner during intercourse.

The two volunteers were a male in his 30's and his wife, both of whom are monogamous and free of any symptoms of genital herpes. The male tested the mixture of zinc gluconate and K-Y Jelly on his forearm first, then on his genitals, using water first and then K-Y Jelly in both locations. They then tested the mixture on the mucous membranes of her vagina by spreading it on the membranes that are close to the outlet of the vaginal canal, in a passive test. It caused no irritation, so it was tested during intercourse. Both people wiped off the excess with a tissue after intercourse, but neither showered or made any effort to wash off the lubricant until the following day. The mixture caused no significant irritation to either person.

EXAMPLE 2: ZINC SULFATE

Zinc sulfate in crystalline form was purchased from Sigma Chemical Company (St. Louis, Missouri). One gram was ground into a fine powder using a mortar and pestle, then 15 ml of K-Y Jelly was added and the two were thoroughly mixed. Although the mixture did not cause any irritation to the male's forearm or genitals, it caused a substantial level of irritation when applied in a passive test to the female's mucous membranes. Therefore, it was not tested during intercourse.

EXAMPLE 3: ZINC CHLORIDE

Zinc chloride in crystalline form was purchased from Sigma Chemical Company (St. Louis, Missouri). One gram was ground into a fine powder using a mortar and pestle, then dissolved in water and applied to the forearm of the male. It caused a burning sensation within a few minutes, and was not tested further.

EXAMPLE 4: ZINC OXIDE

Zinc oxide ointment (Walgreens Pharmaceutical Laboratories, Chicago, IL) was tested in a genital irritation experiment. Zinc oxide was selected because it is known to cause little or no irritation. An ointment sold under the trade name "Desitin" (Pfizer, Inc., New York City), which contains 40% zinc oxide, has been spread on the buttocks and bottoms of babies for years, to control diaper rash and other skin irritations. Desitin also contains cod liver oil, which gives it a rather strong fish odor that renders it undesirable as a sexual lubricant (except possibly for cats). It is also too viscous to make a suitable sexual lubricant.

Other zinc oxide ointments have also been used on the skin for years; such ointments are often spread on the nose to prevent sunburn. One such ointment, sold at Walgreen's (Walgreen Laboratories, Chicago, IL), contains 20% zinc oxide in an ointment base of white wax, petrolatum, and mineral oil.

In skin irritation tests using 20% zinc oxide ointment, preliminary tests on the forearm or other non-sensitive areas were deemed unnecessary. The male mixed approximately equal portions of 20% zinc oxide ointment (Walgreens) with K-Y Jelly to reduce the viscosity of the zinc ointment. He tested it on himself first to ensure that it didn't cause any irritation, then he and his wife used it as a sexual lubricant during intercourse. Other than being a bit sticky and viscous, it did not cause any significant irritation to either person.

# REFERENCES

Al-Nakib, W., et al, "Prophylaxis and treatment of rhinovirus colds with zinc gluconate lozenges," *J Antimicrob. Chemother. 20*: 893-901 (1987)

Asculai, S.S., et al, *Antimicrob. Agents Chemother. 13*: 686 (1978)

Bach, J.-F., "The multi-faceted zinc dependency of the immune system," *Immunology Today,* pp. 225 et seq., November 1981

Becher, P., *Emulsions: Theory and Practice,* 2nd ed., Amer. Chem. Soc. Monograph #162 (Reinhold Publ., New York, 1965)

Bracha, M. and M.J. Schlesinger, "Inhibition of Sindbis virus replication by zinc ions," *Virology 72*: 272-277 (1976)

Brawner, T.A., et al, "A Combined Chemical-Physical Treatment for Herpes Simplex Lesions in Guinea Pigs," *Arch. Dermatol. Res. 265*: 71-77 (1979)

Brody, I., "Topical treatment of recurrent herpes simplex ... zinc sulphate solution," *Brit. J. Dermatol. 104*: 191-194 (1981)

Douglas, R.M., et al, "Failure of effervescent zinc acetate lozenges to alter the course of upper respiratory tract infections in Australian adults," *Antimicrob. Agents Chemother. 31*: 1263-1265 (1987)

Eby, G.A., et al, "Reduction in duration of common colds by zinc gluconate lozenges in a double-blind study," *Antimicrob. Agents Chemother. 25*: 20-24 (1984)

Eby, G.A., and W.W. Halcomb, "Use of topical zinc to prevent recurrent herpes simplex infection: review of literature and suggested protocols," *Medical Hypotheses 17*: 157-165 (1985)

Fahim, M., et al, "New Treatment for Herpes Simplex Virus Type 2 [Ultrasound and Zinc, Urea, and Tannic Acid Ointment] Part 1--Male Patients," *J. Medicine 9(3)*: 245-264 (1978)

Fahim, M., et al, "New Treatment for Herpes Simplex Virus Type 2. Part 2--Female Patients," *J. Medicine 11(2&3)*: 143-167 (1980)

Fahim, M.S. and Brawner, T.A, Treatment of Genital Herpes Simplex Virus in Male Patients," *Arch. Andrology 4*: 79-85 (1980)

Farr, B.M., et al, Two randomized controlled trials of zinc gluconate lozenge therapy of experimentally induced rhinovirus colds," *Antinicrob. Agents Chemother. 31*: 1183-1187 (1987)

Firpo, E.J., and E.L. Palma, "Inhibition of foot and mouth disease virus and procapsid synthesis by zinc ions," *Arch. Virol. 61*: 175-181 (1979)

Fridlender, B., et al, "Selective inhibition of herpes simplex virus type 1 DNA polymerase by zinc ions," *Virology 84*: 551-554 (1978)

Geist F.C., et al, "In vitro activity of zinc salts against human rhinoviruses," *Antimicrob. Agents Chemother. 31*: 622-624 (1987)

Godfrey, J.C., et al, Letters to the Editor, *Antimicrobial Agents and Chemotherapy 32*: 605-609 (1988)

Gordon, Y.J., et al, "Irreversible inhibition of herpes simplex virus replication in BSC-1 cells by zinc ions," *Antimicrob. Agents Chemother. 8*: 377-380 (1975)

Korant, B.D. and B.E. Butterworth, "Inhibition by zinc of rhinovirus protein cleavage: interaction of zinc with capsid polypeptides," *J. Virol. 18*: 298-306 (1976)

Korant, B.D., et al, "Zinc ions inhibit replication of rhinoviruses," *Nature 248*: 588-590 (1976)

Lieberman, H.A, et al, eds., *Pharmaceutical Dosage Forms: Disperse Systems* (Marcel Dekker, New York, 1988)

Marx, J., "The AIDS Virus Can Take On Many Guises," *Science 241*: 1039-1040 (26 August 1988)

Miller, C.J., et al, "An SIV Model for the Heterosexual Transmission of AIDS," *1989 AAAS Annual Meeting Abstracts* p. 47 (Amer. Assn for the Adv. of Science; 1989)

Mindel, A., Herpes Simplex Virus (Springer Verlager, New York, 1989).

Robinson, J.R. and Lee, V.H., eds., *Controlled Drug Delivery* (Marcel Dekker, New York, 1987)

Sergio, W., "Zinc Salts that may be Effective Against the AIDS Virus HIV," *Medical Hypotheses 26(4)*: 251-253 (1988)

Sharma, R., et al, "Antiviral effect of zinc ions on aphthovirus in BHK-21 cell line," *Acta Virol. 29*: 517 (1985)

Shilts, R., *And The Band Played On,* St. Martin's Press, New York, 1987

Tennican, P.O., et al, "The Diverse Effects of Topical and Systemic Administration of Zinc on the Virulence of Herpes Simplex Genitalis," *Life Sciences 24*: 1877-1884 (1979). Also see *Hospital Practice, January 1979, pp. 44-53.*

Tennican, P., et al, "Topical Zinc in the Treatment of Mice Infected Intravaginally with Herpes Genitalis Virus," *Proc. Soc. Exp. Biol. Med 164*: 593-597 (1980)

Wahba, A, "Topical Application of Zinc Solutions: A New Treatment for Herpes Simplex Infections of the Skin?" *Acta Derm. Venerol. (Stockholm) 60*: 175-177 (1980)

Weiner, R.G., "AIDS and Zinc Deficiency," *J. Amer. Med. Assn 252*: 1409-1410 (1984)

Wormser, G., ed., *AIDS and Other Manifestations of HIV Infection,* Noyes Publications, Park Ridge, NJ, 1987

Zaslavsky, V., "Inhibition of vaccinia virus growth by zinc ions: effects on early RNA and thymidine kinase synthesis," *J. Virology 29*: 405-408 (1979)

## Claims

1. A sexual lubricant which comprises a water-soluble or a hydrophobic carrier substance and a zinc compound effective as an anti-viral agent against herpes simplex virus, and which is non-irritating during sexual intercourse.

2. A lubricant according to Claim 1 wherein the zinc compound is mixed with a hydrophilic gel.

3. A lubricant according to Claim 2 wherein the hydrophilic gel comprises a cellulose derivative mixed with water.

4. A lubricant according to Claim 1, wherein the hydrophobic substance comprises vegetable shortening.

5. A lubricant according to Claim 1, wherein the hydrophobic substance consists of water-soluble droplets mixed with a hydrophobic carrier.

6. A lubricant according to any one of the preceding Claims wherein the zinc compound comprises a zinc salt formed of zinc and a carboxylic acid derivative of a carbohydrate.

7. A lubricant according to Claim 6 wherein the carbohydrate comprises a sugar having at least five carbon atoms.

8. A lubricant according to Claim 7 wherein the zinc compound comprises zinc gluconate.